# EUROPEAN PATENT SPECIFICATION

(11) **EP 1 482 919 B1**
(45) Date of publication and mention of the grant of the patent: **02.05.2007**
(21) Application number: 02710518.8
(22) Date of filing: 25.01.2002
(51) Int. Cl.: A61K 31/155, A61K 31/64, A61P 3/10

(54) **PHARMACEUTICAL COMPOSITION THAT IS USED TO CONTROL BLOOD GLUCOSE IN PATIENTS WITH TYPE 2 DIABETES**
PHARMAZEUTISCHE ZUSAMMENSETZUNG ZUR KONTROLLE DES BLUTZUCKERS BEI PATIENTEN MIT TYP 2 DIABETES
COMPOSITION PHARMACEUTIQUE PERMETTANT DE SURVEILLER LE GLUCOSE DANS LE SANG DE PATIENTS SOUFFRANT DE DIABETE DE TYPE 2

(43) Date of publication of application: 08.12.2004
(73) Proprietor: Laboratorios Silanes, S.A. de C.V., México, D.F. 03100 (MX)
(72) Inventor: LARA OCHOA, José Manuel Francisco, México, D.F. 01900 (MX)
(74) Representative: PROPI, S.L.
(86) International application number: PCT/MX2002/000003
(87) International publication number: WO 2003/061643

(56) References cited:
- WO-A-00/40233
- WO-A-01/32158
- CHARPENTIER G. ET AL: 'Improved glycaemic control by addition of glimepiride to metformin monotherapy in Type 2 diabetic patients' DIABETIC MEDICINE vol. 18, núm. 10, 2001, páginas 828 - 834

## Description

### FIELD OF THE INVENTION

This invention consists of providing a therapeutic combination consisting of sulfonylurea glimepiride and biguanide metformin, both oral hypoglycemics, which, when combined, produce, an additive effect and therefore greater effectiveness in controlling blood glucose levels in patients with Type 2 Diabetes.

### BACKGROUND

The use of Sulphonylureas in treating type 2 diabetes is fully established as an effective mean of controlling hypoglycemia. At the molecular level, Sulphonylureas act on the receptor in β pancreatic cells known as SUR, which, when it is activated, closes an ATP dependent potassium channel, which in turn causes a reduction in potassium intake and in consequence depolarization of the membrane. This in turn causes an increase in the flow of calcium toward the cell' 5 interior, activating the cytoskeleton, which causes translocation of secretory granules, thereby releasing insulin by exocytosis.

Another treatment whose use has spread recently is with Biguanide Metformin, which acts effectively not only to control hypoglycemia but also in its prevention. Metformin has a different mechanism for action from Sulphonylureas, increasing insulin sensitivity in hepatic and peripheral tissue (mainly muscular tissues). Metformin inhibits gluconeogenesis and hepatic glycogenolisis. At the cellular level, heightened insulin sensitivity is explained by the increased activity it induces in the tyrosine kinase post-receptor and the resulting increase in the number and activity of GLUT4 transporters.

However, around 75% of type 2 diabetes patients treated with Sulphonylureas do not succeed in bringing their glucose level to the desired values, and need to complement their treatment with a second oral agent. Also, most patients with single drug treatment using Sulphonylureas after a certain number of years require an additional drug that contributes to their control therapy in order to achieve a suitable level of glycemic control. This loss of effectiveness is attributed to various causes, which are not yet well established, such as the supposition that gradual deterioration of the pancreas renders it unable to maintain an exacerbated insulin excretion rate for a long period of time due to constant, long-term stimulation caused by Sulfonylurea therapy. However, contrary to this explanation, Metformin therapy, which does not act by over stimulating β cells, also presents lack of response after prolonged use, which would be contradictory to the explanation given for the lack of response of Sulfonylureas.

On the other hand it has been found that combining Sulphonylurea and Metformin therapy is more effective than monotherapy with either of the two medications. Thus, its been fully proven that the hypoglycemic action of Metformin is completely additional to that of Sulphonylureas (de Fronzo, RA, Goodman AM Yn, England J. Med. 333,541 (1995).

It has also been reported that when monotherapy with Sulphonylureas does not achieve the desired level, it should not be discontinued and replaced by Metformin monotherapy, as this will not lower glucose levels in plasma below the values observed with Sulfonylurea monotherapy (Rosenstock J, Samols E, Muchmore D B, Sheneider J. Diabetes Case 19, 1994 (1996); Gasber AJ, Duncan TG, Goodman AM, Mills DJ, Rohtf JL, AMER J. Med. 103,491 (1997).

It is generally recognized that, because Diabetes Mellitus is a progressive disease, patients with good initial response to oral agents will eventually require a second medication to achieve the desired glycemic control. As we have mentioned, adding Metformin to Sulfonylurea therapy or vice-versa produces an additive response, not only to the reduction in glucose, but also to the reduction in lipids (Hermann LS Schersten B, Bitzen PO, Kjellstrom T, Lindgarde F, Melander A. diabetes Case, 17, 1100 (1994).

Exist several reports and patents on the combined use of the sulfonylurea glibenclamide with the biguanide metformine, among them we have the following:
1. Patent WO 97/17975 of May 27, 1997;
2. Vigneri et al., Diabetes and Metabolism, 17, 232-234 (1991);
3. Higginbotham et al., Med. J. Austr., 154-156 (1979)
4. Patent application US 09/353,141;
5. Patent WO 01/32,158 of November 13, 1999 and
6. Patent US 6,303,146 B1 of October 16, 2001.
7. G. Charpentier et al., Diabetic Medicine, 18 828-834 (2001)

### DESCRIPTION OF THE INVENTION

While there are studies and antecedents for Sulfonylurea gliburide in combination with Metformin, use of Glimepiride in this combination has not been documented to date. Based on studies, we know that Sulphonylureas differ from one another in their insulin release response speed and in the degree of suppression of hepatic glucose achieved (Groop L.m, Luzzzl L, Melander A, Groop PW Ratheises K, Sinonson DC, Diabeters 36, 1320 (1987). As we have been able to establish in this invention, this difference in response can be potentiated by combining Sulphonylureas with Metformin. It has even been reported that the combination of Metformin with a Sulphonylurea as gliburide can have a synergetic effect, given that the two agents act to improve glucose tolerance by different and complementary mechanisms. Thus, we find (Physicians Desk Reference 54^{th} Edition, 2000, page 1349) that the combination of Metformin and gliburide acts synergically by reducing glucose in plasma between meals, glucose in post-prandial plasma and glucosylated hemoglobin to levels of - 63 mg/dL -65 mg/dL and -1.7% respectively. Comparing these results with gliburide monotreatment, the net differences with the combined treatment were +13 mg/dL, -3 mg/dL and +0.2% respectively, and with Metformin monotreatment were - 0.9 mg/dL-1.8 mg/dL and -0.2% respectively.

Combined Glimepiride and Metformin therapy has been suggested previously in the literature (Physicians Desk Reference, 54^{th} Edition, 2000. Page 1349). However, no data are provided on its advantages or appropriate dosages; no clinical studies are cited, and to the contrary, they warn of its supposed risk of hypoglycemia due to concomitant use of the medication Amaryl^{®} (a trademark registered in Mexico, the USA and other countries) and Metformin.

Needless to say, this bibliography does not suggest the possibility of development and use of a pharmaceutical composition with a combination of Glimepiride and Metformin, which, in this invention, we have found to offer unexpected advantages.

The purpose of this invention is to provide a pharmaceutical composition consisting of Glimepiride Sulfonylurea and Biguanide Metformin as its clorhydrate salt, and prove that oral hypoglycemic therapy combining Glimepiride and Metformin in a single pharmaceutical form is more effective and just as safe as monotherapy with the same medications in patients with uncontrolled type 2 diabetes mellitus.

To demonstrate the above, a random, blind, double clinical study was conducted with a universe of 30 patients with uncontrolled type 2 diabetes mellitus who receive monotherapy with Sulphonylureas or Biguanides per group.

### Criteria for inclusion were as follows:

1. Body mass index ≥ 27 kg/m²
2. Age 40 to 65
3. Capacity for deglutition
4. Voluntary consent

### Criteria for exclusion were as follows:

1. Pregnancy
2. Insulin treatment
3. Personal background of systemic diseases such as:
   a) Cardiac insufficiency
   b) Hepatic or chronic hepatopathic insufficiency
   c) Renal insufficiency.
4. Background of significant chronic complications with type 2 diabetes mellitus:
   a) Renal insufficiency
   b) Ischemic cardiopathy
   c) Cerebral vascular disease
   d) Visceral neuropathy.
5. Background of short-term terminal diseases, such as:
   a) Cancer
   b) HIV
6.Therapy with medications that present pharmacological interaction with Glimepiride or Metformin, such as acetazolamide, nicotinic acid, para-aminosalicylic acid, non-steroid anti-inflammatory analgesics, histamine no. 2 antagonists, barbiturates, cyclophosphamide, clonidine, cloramphenicol, cumarinics, disopyramide, epinephrine, statins, fenfluramine, phenothiazine, fibrates, fluoxetine, guanethidine, steroid hormones, ifosfamide, monoamine oxidase inhibitors, laxatives, miconazole, quinolones, reserpine, rifampicine, sulfamides, and tetracycline.
7. Known intolerance or allergies to Sulphonylureas or Biguanides.

### Criteria for exclusion from the therapy, but not from the statistical analysis:

1. presence of severe hypoglycemia at the maximum dosages used in the study
2. Presence of severe hypoglycemia at the minimum dosages used in the study
3. Presence of intolerable undesirable effects with any of the medications used in the study.
4. Failure to follow the medical treatment indicated.
5. Failure to attend scheduled visits.
6. Intercurrent illnesses or accidents that warrant hospitalization.
7. Administration during the study of medications with pharmacological interaction with Metformin Glimepiride.
8. Voluntary withdrawal from the study.

### The variables studied were as follows:

### Dependent variables:

a) Glycemia between meals
b) Glucosilated hemoglobin
c) β cell function
d) Insulin resistance
e) Metabolic profile

### Independent variable:

a) Hypoglycemic therapy

### Intervening variables:

a) Age
b) Sex
c) Body mass index
d) Evolution time of diabetes

### Definitions:

Levels of glycemia between meals > 139 mg/dL are defined as type 2 diabetes mellitus.
Severe hypoglycemia: Glycemia between meals > 260 mg/dL
Severe hypoglycemia: Glycemia < 60 mg/dL
Non-adherence: Medication absorption <80%
Absence: Missed appointment on > 1 occasion.

### Procedure:

1. Identification, clinical history and selection of participants
2. Clinical measurements and basal measurement of glycemia between meals, glucosilated hemoglobin, total cholesterol, cholesterol of high density lipoproteins, triglycerides, creatinine, uric acid, glutemic-oxaloacetic transaminase, glutemic- pyruvic transaminase, lactic dehydrogenase, alkaline and insulin phosphatase.
3. Random assignment of patients to each group and pharmacological intervention in accordance with concentration of basal glycemia:

| a) Glimepiride (2 mg tablets) | |
|---|---|
| Glycemia 140-180 mg/dl 1 mg | ½ tablet |
| Glycemia 181-220 mg/dl 2 mg | 1 tablet |
| Glycemia 221-260 mg/dl 4 mg | 2 tablets |

| b) Metformin (1000 mg tablets) | |
|---|---|
| Glycemia 140-180 mg/dl 500 mg | ½ tablet |
| Glycemia 181-220 mg/dl 1000 mg | 1 tablet |
| Glycemia 221-260 mg/dl 2000 mg | 2 tablets |

| c) Glimepiride/Metformin (2/1000 mg tablets) | |
|---|---|
| 140-180 mg/dl 1/500 mg | ½ tablet |

4. Clinical evaluation 30 and 60 days after beginning the study, measuring glycemia between meals and lactic dehydrogenase.
5. Final clinical evaluations 90 days after the study begins, measuring glycemia between meals, glucosilated hemoglobin, total cholesterol, high density lipoprotein cholesterol, triglyceride, creatinine, uric acid, glutamic-oxaloacetic transaminase, glutamic- pyruvic transaminase, lactic dehydrogenase, alkaline and insulin phosphatase.
6. Undesirable effects were reported on a special record sheet, specifying each of the clinical manifestations considered probable, possible or directly related to the use of the drugs ingested.

The results obtained show that combined Metformin and glimeripide therapy was significantly more effective in controlling glucosylated hemoglobin levels, post-prandial blood glucose levels and blood glucose levels between meals than single-drug treatment with Glimepiride or Metformin alone. The results obtained are shown below:

| | Combination | Glimepiride | Metformin |
|---|---|---|---|
| Glucosylated hemoglobin HbA_{1c} | -0.70 | +0.25 | +0.06 |
| Blood glucose between meals | -1.77 | +0.68 | +0.75 |
| Post-prandial blood glucose | -2.7 | +0.99 | +1.08 |

What is extraordinary about the values obtained is that monotherapy with either Glimepiride or Metformin has a similar effect in raising glucose levels, while treatment with the combination clearly shows a beneficial effect, which highlights its importance.

The previous combinations used in the clinical study can be illustrated by means of the following examples:

### Example 1

A pharmaceutical composition is prepared consisting of 500 mg of Metformin Clorhidrate and 1 mg of Glimepiride, adding the following excipients:

| | |
|---|---|
| Microcrystalline Cellulose PH 101 | 39.20 |
| Colloidal Dioxide of Silica | 1.80 |
| Polyvidone K-90 | 18.00 |
| Sodium Croscarmelose | 12.00 |
| Magnesium stereate | 3.00 |
| Clear Opadray YS-1-7006 | 5.00 |
| Purified water | 0.204 |

This pharmaceutical composition was used for the aforementioned clinical tests.

### Example 2

A pharmaceutical composition is prepared consisting of 500 mg of Metformin Clorhydrate and 2 mg of Glimepiride, adding the following excipients:

| | |
|---|---|
| Microcrystalline Cellulose PH 101 | 38.20 |
| Colloidal Dioxide of Silica | 1.80 |
| Polyvidone K-90 | 18.00 |
| Sodium Croscarmelose | 12.00 |
| Magnesium stereate | 3.00 |
| clear Opadray YS-1-7006 | 5.00 |
| Purified water | 0.204 |

This pharmaceutical composition was used for the aforementioned clinical tests.

### Example 3

A pharmaceutical composition is prepared consisting of 1000 mg of Metformin Clorhidrate and 2 mg of Glimepiride, adding the following excipients:

| | |
|---|---|
| Microcrystalline Cellulose PH 101 | 78.40 mg |
| Colloidal Dioxide of Silica | 3.60 mg |
| Polyvidone K-90 | 36.00 mg |
| Sodium Croscarmelose | 24.00 mg |
| Magnesium stereate | 6.00 mg |
| Clear Opadray YS-1-7006 | 6.25 mg |
| Purified water | 0.345 ml |

This pharmaceutical composition was used for the aforementioned clinical tests.

### Example 4

A pharmaceutical composition is prepared consisting of 1000 mg of Metformin Clorhidrate and 4 mg of Glimepiride, adding the following excipients:

| | |
|---|---|
| Microcrystalline Cellulose PH 101 | 76.40 mg |
| Colloidal Dioxide of Silica | 3.60 mg |
| Polyvidone K-90 | 36.00 mg |
| Sodium Croscarmelose | 24.00 mg |
| Magnesium stereate | 6.00 mg |
| Clear Opadray YS-1-7006 | 6.25 mg |
| Purified water | 0.345 ml |

This pharmaceutical composition was used for the aforementioned clinical tests.

## Claims

1. A pharmaceutical composition to control blood glucose levels in patients with type 2 diabetes **characterized in that** it comprises at least the following compounds in the followings dosages in a single pharmaceutical form:
| | |
|---|---|
| a. Glimepiride | 1-4 mg |
| b. Metformin | 500-1000 mg |

2. Pharmaceutical composition to control blood glucose levels in patients with type 2 diabetes according to claim 1 **characterized in that** it comprises metformin and/or any other salt of metformin of the group consisting of chlorhydrate, succinate and fumarate.

3. Pharmaceutical composition according to claims 1 and 2 **characterized in that** it preferably comprises 500 mg of metformin chlorhydrate and 1 mg of glimepiride.

4. Pharmaceutical composition according to claims 1 and 2 **characterized in that** it preferably comprises 500 mg of metformin chlorhydrate and 2 mg of glimepiride.

5. Pharmaceutical composition according to claims 1 and 2 **characterized in that** it preferably comprises 1000 mg of metformin chlorhydrate and 2 mg of glimepiride.

6. Pharmaceutical composition according to claims 1 and 2 **characterized in that** it preferably comprises 1000 mg of metformin chlorhydrate and 4 mg of glimepiride.

7. Pharmaceutical composition according to claims 1 and 2 **characterized in that** it preferably comprises 500 mg of metformin chlorhydrate and 4 mg of glimepiride.

8. Use of pharmaceutical composition according to any of claims 1 to 7 for the preparation of a medicament for controlling with additive effect blood glucose levels in patients with type 2 diabetes.

## Patentansprüche

1. Eine pharmazeutische Zusammensetzung zur Regulation des Blutglucosespiegels bei Patienten mit Typ-2-Diabetes, **dadurch gekennzeichnet, dass** sie zumindest die folgenden Verbindungen in den folgenden Dosierungen in einer einzigen pharmazeutischen Form umfasst:
| | |
|---|---|
| a. Glimepirid | 1 - 4 mg |
| b. Metformin | 500 - 1000 mg |

2. Pharmazeutische Zusammensetzung zur Regulation des Blutglucosespiegels bei Patienten mit Typ-2-Diabetes nach Anspruch 1, **dadurch gekennzeichnet, dass** sie Metformin und/oder ein anderes Salz von Metformin aus der aus Hydrochlorid, Succinat und Fumarat bestehenden Gruppe umfasst.

3. Pharmazeutische Zusammensetzung nach den Ansprüchen 1 und 2, **dadurch gekennzeichnet, dass** sie vorzugsweise 500 mg Metforminhydrochlorid und 1 mg Glimepirid umfasst.

4. Pharmazeutische Zusammensetzung nach den Ansprüchen 1 und 2, **dadurch gekennzeichnet, dass** sie vorzugsweise 500 mg Metforminhydrochlorid und 2 mg Glimepirid umfasst.

5. Pharmazeutische Zusammensetzung nach den Ansprüchen 1 und 2, **dadurch gekennzeichnet, dass** sie vorzugsweise 1000 mg Metforminhydrochlorid und 2 mg Glimepirid umfasst.

6. Pharmazeutische Zusammensetzung nach den Ansprüchen 1 und 2, **dadurch gekennzeichnet, dass** sie vorzugsweise 1000 mg Metforminhydrochlorid und 4 mg Glimepirid umfasst.

7. Pharmazeutische Zusammensetzung nach den Ansprüchen 1 und 2, **dadurch gekennzeichnet, dass** sie vorzugsweise 500 mg Metforminhydrochlorid und 4 mg Glimepirid umfasst.

8. Verwendung einer pharmazeutischen Zusammensetzung nach einem der Ansprüche 1 bis 7 zur Herstellung eines Medikaments zur Regulation mit Additiveffekt des Blutglucosespiegels bei Patienten mit Typ-2-Diabetes.

## Revendications

1. Une composition pharmaceutique destinée au contrôle des niveaux de glucose dans le sang chez les patients atteints de diabète de type 2, **caractérisée en ce qu'**elle comprend au moins les composés suivants dans les dosages indiqués ci-dessous sous une forme pharmaceutique unique :
| | |
|---|---|
| a. Glimépiride | 1 - 4 mg |
| b. Metformine | 500 - 1000 mg |

2. Composition pharmaceutique destinée au contrôle des niveaux de glucose dans le sang chez les patients atteints de diabète de type 2 selon la revendication 1, **caractérisée en ce qu'**elle comprend de la metformine et/ou tout autre sel de metformine du groupe comprenant chlorhydrate, succinate et fumarate.

3. Composition pharmaceutique selon les revendications 1 et 2, **caractérisée en ce qu'**elle comprend de préférence 500 mg de chlorhydrate de metformine et 1 mg de glimépiride.

4. Composition pharmaceutique selon les revendications 1 et 2, **caractérisée en ce qu'**elle comprend de préférence 500 mg de chlorhydrate de metformine et 2 mg de glimépiride.

5. Composition pharmaceutique selon les revendications 1 et 2, **caractérisée en ce qu'**elle comprend de préférence 1000 mg de chlorhydrate de metformine et 2 mg de glimépiride.

6. Composition pharmaceutique selon les revendications 1 et 2, **caractérisée en ce qu'**elle comprend de préférence 1000 mg de chlorhydrate de metformine et 4 mg de glimépiride.

7. Composition pharmaceutique selon les revendications 1 et 2, **caractérisée en ce qu'**elle comprend de préférence 500 mg de chlorhydrate de metformine et 4 mg de glimépiride.

8. Utilisation de la composition pharmaceutique selon quelconque des revendications 1 à 7 pour la préparation d'un médicament destiné au contrôle avec effet additif des niveaux de glucose dans le sang chez les patients atteints de diabète de type 2.
